# EUROPÄISCHE PATENTSCHRIFT

(11) **EP 0 514 426 B1**
(45) Veröffentlichungstag und Bekanntmachung des Hinweises auf die Patenterteilung: **12.07.1995**
(21) Anmeldenummer: 91903420.7
(22) Anmeldetag: 31.01.1991
(51) Int. Cl.: A61L 2/26, A61L 2/12, A61J 9/00

(54) **VERPACKUNGS-, TRANSPORT- UND DESINFEKTIONSVORRICHTUNG**
DEVICE FOR PACKAGING, TRANSPORTING AND DISINFECTING
DISPOSITIF POUR L'EMBALLAGE, LE TRANSPORT ET LA DESINFECTION

(30) Priorität: 09.02.1990 AT 291/90
(43) Veröffentlichungstag der Anmeldung: 25.11.1992
(73) Patentinhaber: MAM BABYARTIKEL GESELLSCHAFT M.B.H., A-1160 Wien (AT)
(72) Erfinder: RÖHRIG, Peter, A-1160 Wien (AT)
(74) Vertreter: Weinzinger, Arnulf, Dipl.-Ing.
(86) Internationale Anmeldenummer: AT9100014
(87) Internationale Veröffentlichungsnummer: WO9112028

(56) Entgegenhaltungen:
- DE-A- 2 839 219
- DE-A- 3 740 798
- FR-A- 2 090 487

## Beschreibung

Die Erfindung betrifft eine Verpackungs-, Transport- und Desinfektionsvorrichtung für Babyartikel und dergl. Gegenstände, wie z.B. Saugflaschen, Sauger, Schnuller.

Weiters bezieht sich die Erfindung auf ein Verfahren zur Desinfektion von Babyartikeln und dergl. Gegenständen, wie z.B. Saugflaschen, Sauger, Schnuller, unter Verwendung einer solchen Vorrichtung.

### Zugrundeliegender Stand der Technik

Babyartikel, wie z.B. Saugflaschen mit dazugehörigen Saugern, ferner Schnuller, Beißringe usw., werden derzeit in den verschiedensten Verpackungen zum Verkauf ausgestellt und ausgeliefert, und es besteht ein Bedarf an einer hygienischen und verkaufstechnischen Anforderungen entsprechenden Verpackung für die Auslieferung vom Hersteller zum Verkäufer sowie für die Präsentation in Verkaufsgeschäften.

Weiters bereitet es derzeit Schwierigkeiten, Saugflaschen in mit einer Babynahrung gefülltem Zustand bequem und sicher transportieren zu können, um ein Kind an einem anderen Ort als zu Hause füttern zu können. Ein weiteres Problem liegt sodann darin, daß die genannten Gegenstände in periodischen Abständen, Saugflaschen und Sauger insbesondere vor jedem Gebrauch, in einen weitgehend keimfreien Zustand gebracht werden müssen, wobei dieser Vorgang hier zur Vereinfachung mit dem gängigen Fachausdruck "Desinfektion" umschrieben werden soll. Zu dieser Desinfektion wird üblicherweise ein Auskochen der genannten Gegenstände oder aber ein Einlegen in einem chemischen Sterilisationsbad vorgenommen. Beim Auskochen der Gegenstände werden die Gegenstände, wie Flaschen, Schnuller, Sauger usw., einige Minuten lang in kochendem Wasser belassen. Dieses Auskochen bringt zwar eine gute Desinfektion mit sich, ist jedoch stark materialbelastend für die behandelten Gegenstände, wobei hinzu kommt, daß die notwendige Mindestkochzeit in der Regel aus Sicherheitgründen weit überschritten wird. Auch ist das Hantieren mit den ausgekochten Gegenständen umständlich, wobei eine ausreichende Abkühlung abgewartet werden muß, so daß auch eine Verbrühungsgefahr besteht.

Die chemische Sterilisationsbehandlung ist andererseits vor allem deshalb von Nachteil, da eine verhältnismäßig genaue Dosierung des Sterilisationsmittels für das Sterilisationsbad notwendig ist; weiters muß dieses Sterilisationsmittel vorrätig gehalten werden, wobei der Aufbewahrung wegen der Verwechslungsgefahr besondere Aufmerksamkeit zu widmen ist, was im Hinblick darauf, daß das Sterilisationsbad zumindest einmal täglich angesetzt werden muß, mit erheblichen Unbequemlichkeiten verbunden ist. Überdies ergibt sich durch das Weggießen des Bades eine erhöhte Umweltbelastung.

DE-A-3 740 798 offenbart eine Vorrichtung zur Sterilisation von Babymilchflaschen unter Verwendung von Mikrowellen.

### Zusammenfassung der Erfindung

Es ist nun Aufgabe der Erfindung, eine einzige Vorrichtung für alle vorgenannten Funktionen zu schaffen, so daß diese Vorrichtung eine hygienisch einwandfreie und verkaufstechnisch günstige Verpackung, weiters einen bequemen und sicheren Transport von gefüllten Saugflaschen sowie auch eine einfache und problemlose Desinfektion der genannten Gegenstände ermöglicht. Weiters soll die Erfindung ein einfaches und bequemes Verfahren zur Desinfektion der genannten Gegenstände unter Verwendung dieser Vorrichtung vorsehen.

Die erfindungsgemäße Verpackungs-, Transport- und Desinfektionsvorrichtung ist gekennzeichnet durch einen aus einem mikrowellentauglichen Kunststoff, wie Polykarbonat, bestehenden Behälter mit Deckel, wobei der Deckel, z.B. mittels Schnapp-oder Knebelverbindungen, dicht mit dem oben offenen Behälterkörper verbindbar ist, der Behälterkörper an zwei einander gegenüberliegenden Seiten mit angelenkten Handgriffen versehen ist, von denen zumindest einer an den Behälterkörper anklappbar ist, um eine Behälter-Standfläche zusätzlich zum Boden des Behälters vorzusehen, im Deckel zumindest eine Druckausgleichsöffnung vorgesehen ist und der Behälter mit einer von außen sichtbaren Temperaturanzeige ausgerüstet ist. Auf diese Weise wird eine dicht schließende Verpackung für die genannten Gegenstände, z.B. für zwei Saugflaschen samt Zubehör, erhalten, wobei diese Verpackung zur Lagerung sowie zur Präsentation in Geschäftslokalen wie auch als Transportverpackung, und zwar insbesondere auch für gefüllte Saugflaschen, mit Vorteil verwendbar ist. Dabei ist insbesondere von Vorteil, daß die Handgriffe ein bequemes Tragen der Vorrichtung ermöglichen, wobei ein Handgriff zum Erfassen ausgeklappt wird, während der gegenüberliegende Handgriff am Behälterkörper angeklappt bleibt und so eine zusätzliche Standfläche zum Abstellen des zuvor mit dem anderen Handgriff getragenen Behälters ermöglicht. Diese aufrechte Position des Behälters kann im übrigen auch für die Zurschaustellung beim Verkauf von Vorteil sein. Sodann ermöglicht die wie angegeben ausgebildete Vorrichtung auch auf einfache Weise eine Desinfektion der jeweiligen Gegenstände, wobei die Vorrichtung mit diesen Gegenständen beispielsweise in einem Mikrowellenherd angeordnet wird.

In diesem Zusammenhang sieht die Erfindung ein Verfahren vor, das dadurch gekennzeichnet ist, daß in den Behälterkörper Wasser eingebracht wird und die zu desinfizierenden Gegenstände zumindest im wesentlichen oberhalb des Niveaus des eingebrachten Wassers angebracht werden, und daß nach Schließen des Behälters mit dem Deckel die Vorrichtung einem Mikrowellenfeld ausgesetzt wird, um die Gegenstände durch eine Behandlung im dabei entstehenden Wasserdampf zu desinfizieren. Dabei wird davon ausgegangen, daß heute bereits in sehr vielen Haushalten Mikrowellenherde vorhanden sind, in denen Wasser bzw. wasserhältige Nahrungsmittel durch Einwirkung eines Mikrowellenfeldes erhitzt werden können. Dieser Umstand wird erfindungsgemäß zur Desinfektion der Saugflaschen, Sauger, Schnuller und dergl. Gegenstände ausgenutzt, wobei die Desinfektion im Vergleich zu dem eingangs erwähnten Auskochen wesentlich rascher und vergleichsweise gefahrlos sowie materialschonend durchgeführt werden kann. Ferner werden die Nachteile vermieden, die dem ebenfalls eingangs erwähnten Sterilisationsbad anhaften, insbesondere auch jener, daß Sterilisierflüssigkeit an den zu desinfizierenden Gegenständen haften bleibt und zumindest den Geschmack von mit Hilfe der Saugflasche und des Saugers verabreichter Nahrung, aber auch den Geschmack von Schnullern, Beißringen und dergl. beeinträchtigt. Für diese erfindungsgemäße Desinfektion im Mikrowellenfeld, in einer Dampfatmosphäre, wird ein und dieselbe Vorrichtung verwendet, die wie vorstehend erwähnt auch als Verkaufs-, Präsentations- und Transportbehälter verwendet wird.

Für die Abtötung der häufigsten Keime bei den Gegenständen der hier in Rede stehenden Art (z.B. streptococcus faecalis, staphylococcus epidermis, candida albicans) ist es in der Regel erforderlich, daß die zu desinfizierenden Gegenstände zirka ein Minute lang mindestens auf 80°C erwärmt werden und sich dabei in einer Atmosphäre von gesättigtem Wasserdampf, der auf Seehöhe eine Temperatur von 100°C hat, befinden. Die Erreichung dieser Bedingungen bei Anwendung der Erfindung hängt natürlich von der Leistung des verwendeten Mikrowellenherds, aber auch von der Ausgangstemperatur der in ihn eingebrachten Teile sowie von der Masse der zu desinfizierenden Gegenstände ab. Die hiefür beeinflußbaren Parameter sind die am Mikrowellenherd einstellbaren Größen, nämlich Leistung und Zeitdauer der Erhitzung im Mikrowellenfeld. Diese Parameter können leicht empirisch für die ungünstigsten Ausgangstemperaturen ermittelt und gegebenenfalls als Sicherheitswerte beispielsweise in einer Gebrauchsanleitung übermittelt werden. Im übrigen ist hier die erfindungsgemäß vorgesehene Temperaturanzeige von Bedeutung, mit der überprüft werden kann, ob die erforderliche Temperatur über die notwendige Zeitdauer erreicht wurde.

Bei der erwähnten Desinfektion werden je nach Art der Gegenstände, die zu desinfizieren sind, diese Gegenstände beispielsweise direkt auf den Behälterboden gelegt, wobei sie teilweise in das in den Behälter eingebrachte Wasser eintauchen können, oder sie werden vorzugsweise unter Vorsehen irgenwelcher Zwischenlagen, die sie im Abstand vom Behälterboden halten, in den Behälter eingelegt. Diese Zwischenlagen können dabei an sich irgendwelche im Haushalt vorhandene Kunststoffteile sein, jedoch ist es erfindungsgemäß ganz allgemein von Vorteil, wenn im Behälter am Boden Distanzelemente zum Abstützen der zu desinfizierenden Gegenstände in Abstand vom Behälterboden vorgesehen sind.

Diese Distanzelemente können dabei im Prinzip auch durch mit dem Boden einteilige, vom Boden hochstehende Vorsprünge gebildet sein. Im Hinblick auf die anzustrebende gute Reinigungsmöglichkeit sowie darauf, daß bei der beschriebenen Desinfektion beim Verdampfen von Wasser Kalkablagerungen entstehen können, ist es jedoch erfindungsgemäß besonders günstig, wenn die Distanzelemente durch einen herausnehmbaren, z.B. gitterartigen Rost gebildet sind. Dabei ist es weiters für die Verdampfung des Wassers beim Desinfizieren vorteilhaft, wenn sich der Rost mit randseitigen Füßen am Boden des Behälterkörpers abstützt. Aus Stabilitätsgründen ist es hier ferner von Vorteil, wenn der Rost im Bereich der Mitte des Bodens des Behälterkörpers auf einer hochgezogenen Einbuchtung des Bodens aufliegt.

Zur Lagefixierung des Rostes im Behälter, etwa beim Transport, ist es auch vorteilhaft, wenn der Rost zumindest an zwei einander gegenüberliegenden Seiten an seinem Rand elastisch verformbare Ausbuchtungen aufweist, mit denen er unter federndem Druck an den benachbarten Seitenwänden des Behälterkörpers anliegt.

Um das Einsetzen des Rostes in den Behälter möglichst einfach zu gestalten, ist es auch günstig, wenn der Rost zumindest im wesentlichen symmetrisch ausgebildet ist, wobei es insbesondere zweckmäßig sein kann, eine Symmetrie nicht nur hinsichtlich zweier zueinander senkrechter, den Rost bzw. dessen Hauptebene senkrecht schneidender Mittenebenen vorzusehen, sondern auch hinsichtlich der Hauptmittelebene des Rostes selbst, so daß Oberseite und Unterseite des Rostes gleich ausgebildet sind.

Im Hinblick auf die Funktion der Präsentation in Verkaufslokalen ebenso wie auch jene der Verpackung für den Transport, wo ebenfalls der Inhalt der Vorrichtung möglichst mit einem Blick erfaßt werden können soll, sowie vor allem im Hinblick auf die Desinfektionsfunktion, wobei die Temperaturanzeige für die Überprüfung des Desinfektionsvorganges nach Möglichkeit im Behälterinneren anzubringen ist, ist es erfindungsgemäß auch von Vorteil, wenn zumindest der Deckel aus klarem Polykarbonat besteht, so daß die enthaltenen Gegenstände bzw. eine innenliegende Temperaturanzeige problemlos von außen ersichtlich sind.

Weiters hat es sich für die angegebene Dreifachfunktion auch als besonders günstig erwiesen, wenn der Behälter annähernd quaderförmig ausgebildet ist. Bei dieser Form ist eine einfache, gedrängte, platzsparende Lagerung mehrerer Behälter über- und nebeneinander ebenso in vorteilhafter Weise möglich wie die Mitnahme des Behälters etwa bei Tagesausflügen und inbesondere auch seine Anbringung in einem Mikrowellenherd zwecks Desinfektion von Gegenständen.

Für die angestrebten Zwecke, insbesondere hinsichtlich Verpackung und Transport, ist es sodann auch günstig, wenn der Behälter in ungefähr zwei Drittel seiner Höhe in den eigentlichen Behälterkörper und den aufgesetzten Deckel unterteilt ist, d.h. der Deckel erstreckt sich über ungefähr ein Drittel der gesamten Behälterhöhe und der Behälterkörper über zwei Drittel der Behälterhöhe.

Die Handgriffe, die wie erwähnt im angeklappten Zustand eine zusätzliche Behälterstandfläche ermöglichen, können im Prinzip auf irgendeine an sich herkömmliche Weise an den Behälterwänden angelenkt sein. Eine herstellungsmäßig günstige und dabei ausreichen und stabile Befestigung wird dann ermöglicht, wenn an den einander gegenüberliegenden Seiten des Behälterkörpers Scharniere für die Handgriffe angeformt sind.

Für die stabile Fixierung der Handgriffe sowie eine möglichst einfache Montage ist es hier auch günstig, wenn für jeden Handgriff zwei Scharnierblöcke in Abstand voneinander am Behälterkörper angeformt sind und diese Scharnierblöcke zapfenartige Angeln des jeweiligen bügelartigen Handgriffs schwenkbar aufnehmen.

Zur Erzielung einer stabilen zusätzlichen Standfläche ist es auch von Vorteil, wenn die Scharniere jeweils direkt unterhalb des die obere Öffnung des Behälterkörpers begrenzenden Randes vorgesehen sind. Zu beachten ist hiebei jedoch sicherlich, daß der Anlenkpunkt der Handgriffe nicht zu weit außerhalb der Mitte der Behälterhöhe sein sollte, um ein Schrägstellen des Behälters beim Tragen möglichst zu vermeiden.

Um ein ungewolltes Hin- und Herschwenken des Behälters beim Tragen hintanzuhalten und auch ein ungewünschtes Wegklappen der angeklappten Handgriffe von der Behälterwand, etwa beim Abstellen des Behälters auf der zusätzlichen Standfläche, zu verhindern, ist es auch zweckmäßig vorzusehen, daß die Handgriffe in diesen zwei spezifischen Stellungen, nämlich der angeklappten Stellung einerseits wie der um 90° hiezu herausgeschwenkten Stellung, festgehalten werden. Demgemäß ist es von Vorteil, wenn die Handgriffe in den Scharnieren zwischen zwei Raststellungen, einer an den Behälterkörper angeklappten Stellung und einer rechtwinkelig hievon abstehenden Stellung, verschwenkbar gelagert sind. Andererseits kann hier auch vorgesehen werden, daß die Handgriffe mit Reibungspassung in den Scharnieren gelagert sind. In beiden Fällen wird zumindest für die erwähnten Stellungen der Handgriffe eine gewisse Lagefixierung erreicht.

Vorzugsweise reichen die Handgriffe in der an den Behälterkörper angeklappten Stellung bis annähernd zum Boden des Behälterkörpers. Auf diese Weise können, abgesehen von einer sicheren zusätzlichen Standfläche, auch ausreichend große Handgriffe zum Erfassen sichergestellt werden.

An sich könnten die Handgriffe in der an den Behälterkörper angeklappten Stellung innerhalb einer Vertiefung in der Behälterwand aufgenommen sein, und die Umrandung dieser Vertiefung würde dann die beschriebene zusätzliche Behälterstandfläche bilden. Zur Vereinfachung der Konstruktion sowie auch Handhabung ist es jedoch günstig, wenn die Handgriffe selbst in der an den Behälterkörper angeklappten Stellung mit ihrer Außenseite einen Teil der zusätzlichen Behälter-Standfläche bilden.

Vor allem beim Transport von gefüllten Saugflaschen etc. ist es wichtig, daß der Deckel einen dichten Verschluß des Behälters sicherstellt, so daß kein Staub oder dergl. in das Behälterinnere gelangen kann. Zur Erzielung des erforderlichen, insbesondere staubdichten Verschlusses hat es sich dabei erfindungsgemäß als besonders günstig erwiesen, wenn der Behälterkörper und der Deckel mit zueinander passenden, rundum laufenden, nach außen abstehenden Flanschen für ihre dichte Verbindung versehen sind. Dabei können die Flanschen mit den aufeinanderliegenden Flächen, sofern diese ausreichend plan sind, bereits von selbst für den ausreichend dichten Abschluß sorgen; es ist jedoch auch durchaus denkbar, in zumindest einem der Flanschen eine Dichtungsleiste einzulegen oder einen Dichtungsstreifen daran aufzukleben. Überdies können diese Flanschen auch für die Anbringung von irgendwelchen an sich herkömmlichen Verbindungseinrichtungen, wie Schnappverbindungselementen, Rastverbindungselementen oder Knebelverbindungen, benützt werden, um den Deckel ausreichend fest mit dem Behälterkörper zu verbinden.

Für die erwähnte Desinfektionsfunktion hat es sich zur Temperaturüberwachung und damit zur Überwachung des Verlaufes des Desinfektionsvorganges als vorteilhaft erwiesen, wenn als Temperaturanzeige an bzw. in einer Wand des transparenten Deckels innen ein Temperaturanzeigeplättchen angebracht ist. Vorzugsweise ist dabei das Temperaturanzeigeplättchen in eine taschenartige Vertiefung mit übergreifenden Randleisten eingesetzt. Auf diese Weise ist auch gegebenenfalls ein Auswechseln des Temperaturanzeigeplättchens ohne weiteres möglich.

Für eine von außen gut sichtbare Anzeige der im Behälterinneren erreichten Temperatur ist es auch von Vorteil, wenn das Temperaturanzeigeplättchen ein Temperaturindikator auf Flüssigkristallbasis ist. Dabei ist das Erreichen der erforderlichen Desinfektionstemperatur durch entsprechende Verfärbung des Temperaturanzeigeplättchens, wie an sich bekannt, feststellbar. Es sei hier erwähnt, daß solche Anzeigeplättchen oder -streifen auf Flüssigkristallbasis beispielsweise in Spitälern zur Kontrolle von Niedertemperatur-Dampfprozessen in anderem Zusammenhang verwendet werden, und es gibt Anzeigplättchen, die nur in Gegenwart von Wasserdampf arbeiten.

Besonders günstig ist es, wenn das Temperaturanzeigeplättchen eine Zeit-Temperatur-abhängige Charakteristik hat und die Erreichung einer vorgegebenen, für eine einwandfreie Desinfektion notwendigen Temperatur über eine vorgegebene Zeitdauer anzeigt. Ein solches Plättchen, das wie erwähnt vorzugsweise an der Innenseite des transparenten Behälterdeckels angebracht wird, zeigt durch entsprechende Verfärbung, die erst dann auftritt, wenn die vorgeschriebene Temperatur über eine bestimmte, vorgegebene Zeit vorlag, die zufriedenstellende Desinfektion an.

Um das Magnetron des Mirkowellenherdes nicht zu beschädigen, ist es schließlich bei der Desinfektion auch zweckmäßig, wenn die Menge des in den Behälter eingebrachten Wassers ausreichend bemessen wird, so daß ein Wasserrest nach der Dampf-Desinfektionsbehandlung im Behälter bleibt.

Zu erwähnen ist noch, daß an sich bereits ein Erhitzungsgerät, inbesondere zur Desinfektion für Babyflaschen, in der DE-A-36 00 369 vorgeschlagen worden ist. Dabei handelt es sich jedoch um ein Gerät, das ähnlich den sog. Eierkochern aufgebaut ist und im übrigen auch zum Kochen von Eiern verwendbar sein soll. Dieses bekannte Erhitzungsgerät enthält einen eigenen Heizteil, an dessen Oberseite eine Wanne zur Aufnahme von Wasser vorgesehen ist, und auf den ein eigener speziell konstruierter Aufnahmebehälter für die zu desinfizierenden Gegenstände oder aber für die zu kochenden Eier aufgesetzt ist. Dieses bekannte Gerät ist somit verhältnismäßig aufwendig, und es hat auch einen nicht unerheblichen Raumbedarf; überdies sind wegen des eigenen Heizteils entsprechende Sicherheitseinrichtungen, insbesondere gegen Trockenlauf, notwendig, und dieses bekannte Gerät eignet sich auch nicht als Verkaufsverpackung oder Transportvorrichtung. Ein weiterer Nachteil dieses bekannten Erhitzungsgerätes liegt darin, daß wegen der indirekten Erhitzung des eingebrachten Wassers dessen Aufheizen und Verdampfen eine relativ lange Zeit erfordert. Im Gegensatz dazu wird bei der Erhitzung in einem Mikrowellenfeld, wie im Fall der Erfindung, praktisch die gesamte Heizeinergie direkt dem Wasser zugeführt, wodurch ein schnelles Aufheizen und Verdampfen erzielt wird.

Auch sei noch erwähnt, daß aus der EP-A-47 356 bereits eine Vorrichtung zum Sterilisieren von medizinischen Gegenständen bekannt ist, wobei ein flaschenförmiger Behälter, in den die medizinischen Gegenstände eingelegt werden, in seinem nach unten gerichteten Flaschenhals ein Reaktionsmedium, z.B. Wasser oder Alkohol, aufnimmt. Dieser Behälterteil soll in den Brennpunkt eines Reflektors eines Infrarotstrahlers oder aber in den Bereich der höchsten Leistungsdichte eines Mikrowellenstrahlers gebracht werden, um das Reaktionsmedium zu verdampfen. Es handelt sich hierbei ersichtlich um eine sehr spezialisierte Einrichtung, die nicht mit einem im Haushalt vorhandenen Mikrowellenherd betrieben werden kann, und die auch nicht die Mehrfachfunktionen erfüllt, wie sie der erfindungsgemäßen Vorrichtung zugrunde liegen.

### Kurzbeschreibung der Zeichnung

Die Erfindung wird nachstehend anhand von in der Zeichnung veranschaulichten bevorzugten Ausführungsbeispielen, auf die sie jedoch nicht beschränkt sein soll, noch weiter erläutert. Es zeigen:
Fig. 1 eine axonometrische Ansicht einer erfindungsgemäßen Vorrichtung;
Fig. 2 eine Seitenansicht dieser Vorrichtung, wobei die rechte Hälfte im Schnitt gezeigt ist;
Fig. 3 eine Vorderansicht dieser Vorrichtung, teilweise im Schnitt;
Fig. 4 eine Draufsicht auf diese Vorrichtung, mit teilweise weggebrochenem Behälterdeckel;
Fig. 5 in einem Detailschnitt gemäß der Linie V-V in Fig. 4 ein Scharniergelenk für einen Handgriff dieser Vorrichtung;
Fig. 6 einen Detailschnitt gemäß der Linie VI-VI in Fig. 1, zur Veranschaulichung der Anbringung einer Temperaturanzeigeplättchens an der Innenseite des Behälterdeckels;
die Fig. 7 und 8 in Detail-Schnittdarstellungen gegenüber Fig. 2 modifizierte Ausführungsformen der Verbindung des Behälterdeckels mit dem Behälterkörper; und
Fig. 9 einen waagrechten Schnitt durch den Behälterkörper, zur Veranschaulichung einer abgewandelten Ausführungsform des in den Behälterkörper eingelegten Rostes.

### Detaillierte Beschreibung der bevorzugten Ausführungsform

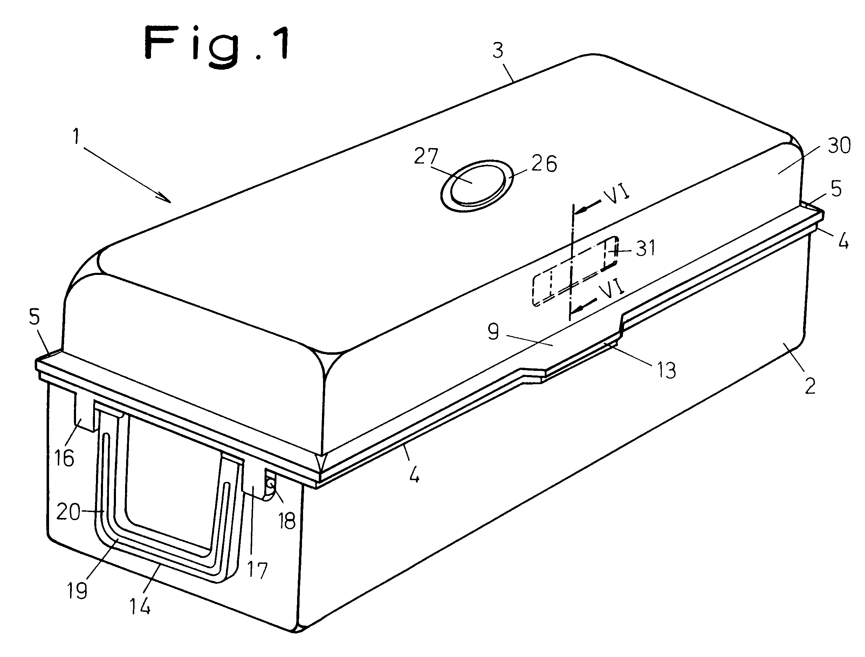

Die insbesondere aus den Fig. 1 bis 4 ersichtliche Verpackungs-, Transport- und Desinfektionsvorrichtung, die derzeit als beste Ausführungsform betrachtet wird, besteht in diesem Ausführungsbeispiel aus einem im wesentlichen quaderförmigen Behälter 1, der in ungefähr zwei Drittel seiner Höhe unterteilt ist, so daß ein ungefähr zwei Drittel der gesamten Behälterhöhe einnehmender unterer Behälterkörper 2 sowie ein sich über ein Drittel der Behälterhöhe erstreckender Deckel 3 vorliegen. Der Behälterkörper 2 und der Deckel 3 bestehen aus einem an sich herkömmlichen mikrowellentauglichen Kunststoffmaterial, wie etwa Polykarbonat, das die für einen Einsatz in einem Mikrowellenfeld geeignete dielektrische Konstante und einen minimalen Wassergehalt hat. Der Behälter 1, insbesondere der Behälterkörper 2, kann leicht eingefärbt sein, wobei jedoch bevorzugt wird, daß der Behälter 1 zumindest durchscheinend ist, um die Zurschaustellung der darin enthaltenen Gegenstände in einem Verkaufslokal nicht zu beeinträchtigen. Der Deckel 3 besteht vorzugsweise aus einem glasklarem Kunststoffmaterial, und zwar nicht nur deshalb, um so eine bessere Einsicht auf die im Behälter 1 enthaltenen Gegenstände von oben zu gewährleisten, sondern vor allem deshalb, um beim Desinfektionsvorgang in einem Mirkowellenherd eine Überprüfung der erreichten Temperatur zu ermöglichen, wie nachstehend noch näher erläutert werden wird.

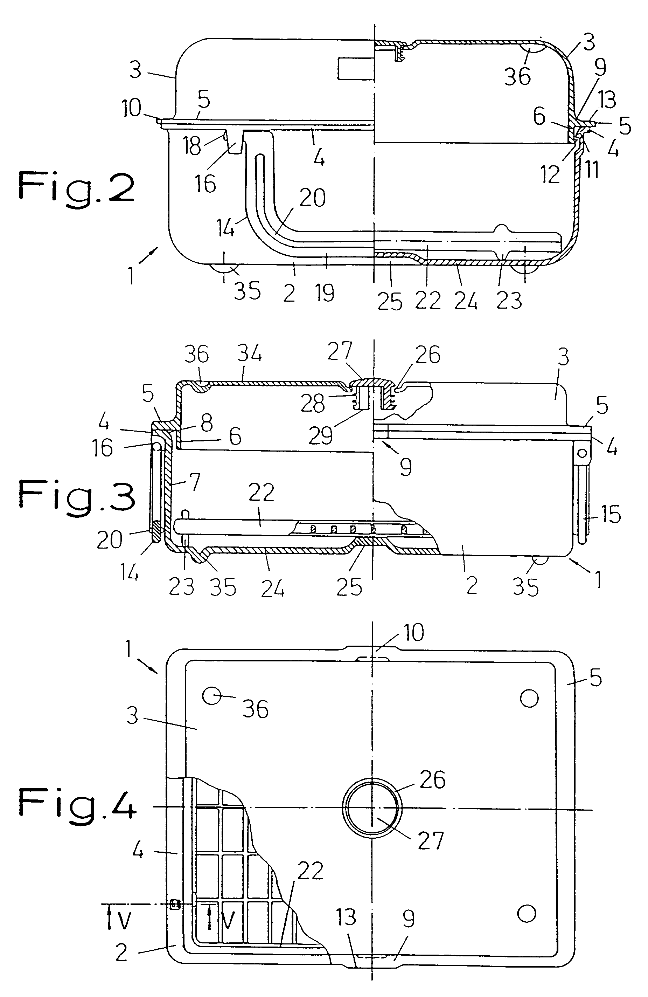

Der Behälterkörper 2 und der Deckel 3 sind bei der Ausführungsform gemäß Fig. 1 bis 4 an ihren beim Schließen des Behälters 1 zusammenzufügenden Rändern mit umlaufenden Randflanschen 4 bzw. 5 versehen, die an den einander zugewandten Seiten plan sind und mit diesen planen Flächen dicht aneinander anliegen, vgl. insbesondere auch die Schnittdarstellungen in Fig. 2 und 3. Wie dabei weiters aus Fig. 2 wie auch aus Fig. 3 ersichtlich ist, besitzt der Deckel 3 innen einen Wandfortsatz 6, der als vertikaler Zentrierfortsatz das Aufsetzen des Deckels 3 auf den Behälterkörper 2 erleichtert. Hiefür wirkt sich auch die am Behälterkörper 2 im Bereich des Übergangs von den vertikalen Wänden, z.B. 7 (Fig. 3), in den horizontalen Flansch 4 vorgesehene Rundung 8 günstig aus.

Ungefähr in der Mitte der beiden Längsseiten des Behälters 1 erfolgt die Verankerung des Deckel 3 am Behälterkörper 2, und zwar in Verbindungsbereichen 9, 10; wie insbesondere aus Fig. 2 ersichtlich ist, ist bei der vorliegenden Ausführungsform eine Schnapp- oder Rastverbindung mit einer einen hakenförmigen Vorsprung 11 am Behälterkörper 2 und einer ihn hintergreifenden Rastnase 12 am Wandfortsatz 6 des Deckels 3 vorgesehen. Der Flansch 5 des Deckels 3 ist in diesen Verbindungsbereichen 9, 10 verbreitert, wie bei 13 in Fig. 1, 2 und 4 ersichtlich ist, um so eine Handhabe zum Öffnen des Behälters 1, d.h. zum Abnehmen des Deckels 3 vom Behälterkörper 2 vorzusehen.

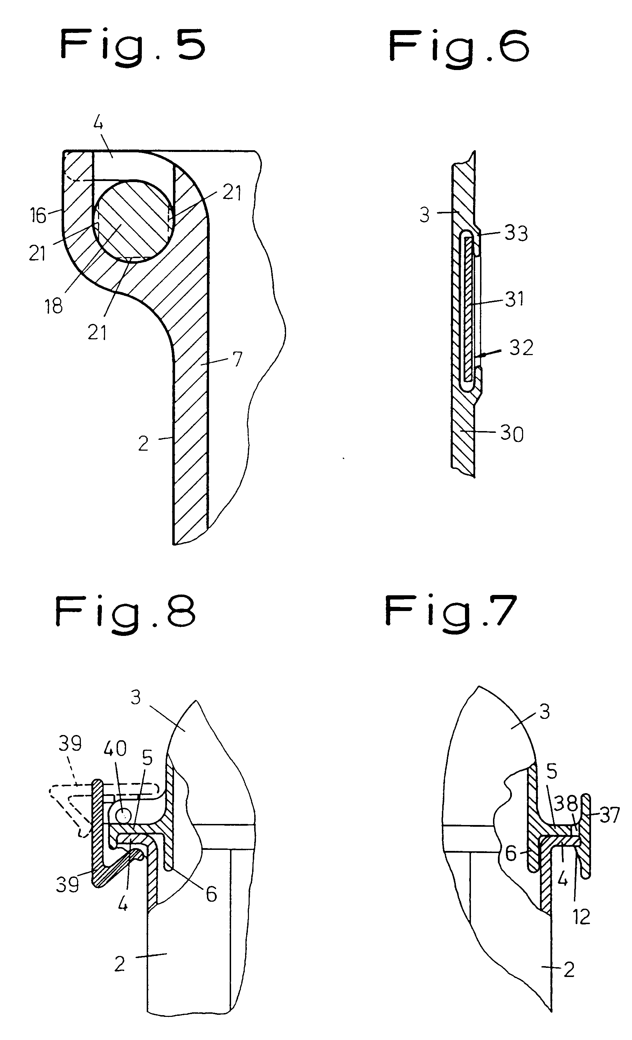

An den beiden einander gegenüberliegenden Schmalseiten des Behälters 1, d.h. an den vertikalen Wänden 7 des Behälterkörpers 2, sind bügelartige Handgriffe 14, 15 am Behälterkörper 2 angelenkt. Im einzelnen sind dabei unmittelbar unterhalb des Flansches 4 des Behälterkörpers 2 für jeden Handgriff 14 bzw. 15 zwei Scharnierblöcke 16, 17 am Behälterkörper 2 angeformt, und in diesen Scharnierblöcken 16, 17 sind die zapfenartigen Angeln 18 (s. auch Fig. 5) der Handgriffe 14, 15 schwenkbar gelagert. Die Handgriffe 14, 15 sind wie erwähnt bügelförmig, d.h. in Ansicht U-förmig, und sie reichen mit ihrem horizontalen Steg 19 bis nahe an den Behälterboden. Die Stärke der Handgriffe 14, 15 ist dabei, gegebenenfalls unter Vorsehen eines entlanglaufenden Wulstes 20 auf beiden Handgriffseiten oder zumindest auf beiden Stegseiten der Handgriffe, derart bemessen, daß diese Handgriffe 14, 15 bzw. deren Wulste 20 zusammen mit den Rändern der Flanschen 4, 5 an den beiden Schmalseiten des Behälters 1 in einer Ebene liegen, wenn die Handgriffe angeklappt sind. Demgemäß definieren diese Handgriffe 14, 15 im angeklappten Zustand zusammen mit den Flanschen 4, 5 eine zusätzliche Behälter-Standfläche, wenn der Behälter in einer von der normalen Abstellposition, die aus Fig. 1 bis 3 ersichtlich ist, abweichenden "Hochkant"stellung abgestellt werden soll, in der er mit seiner Längsrichtung vertikal ausgerichtet ist und sich der eine Handgriff dann oben am Behälter befindet.

Für die Erzielung der erwähnten zusätzlichen Behälter-Standfläche ist es auch von Bedeutung, daß die Handgriffe 14, 15 in der an den Behälterkörper angeklappten Stellung, beispielsweise mit dem innenseitigen Wulst 20, an die Behälterwand 7 außen anliegen, wenn sie mit ihrer gegenüberliegenden Außenseite die zusätzliche Standfläche definieren.

Die Ausbildung der Angeln 18 bzw. der zugehörigen Lagerausnehmungen in den Scharnierblöcke 16, 17 ist derart, daß diese Angeln 18 mit Reibungspassung in den Scharnierblöcken 16, 17 aufgenommen sind, so daß die Handgriffe 14, 15 mehr oder weniger stabil in jeder Schwenklage zwischen den gezeigten angeklappten vertikalen Stellungen und den horizontal ausgeklappen Stellungen festgehalten sind. Dadurch wird beispielsweise das Abstellen des Behälters 1 in der Hochkantstellung erleichtert, da der an der dann unteren Schmalseite des Behälters befindliche Handgriff in der an den Behälterkörper 2 angeklappen Stellung verbleibt. Anstelle der erwähnten Reibungspassung ist es aber jedoch auch möglich, die Angeln 18 der Handgriffe 14, 15 in den zwei Präferenzstellungen der Handgriffe (an die Behälterwand angeklappt bzw. im rechten Winkel dazu ausgeklappt) einschnappen zu lassen, wozu beispielswiese in an sich herkömmlicher Weise entsprechend abgeflachte Bereiche an den Außenflächen der Angeln 18 bzw. in den Lagerausnehmungen der Scharnierblöcke 16, 17 sowie eine ausreichende elastische Verformbarkeit der Scharnierblöcke vorzusehen wären. Eine derartige Abflachung ist beispielsweise in Fig. 5 schematisch mit gestrichelten Linien bei 21 angedeutet.

Eine andere Möglichkeit bestünde darin, für das Einschnappen in den erwähnten zwei Vorzugsstellungen zusammenarbeitende Schnappvorsprünge und- vertiefungen in den aneinander gleitenden Lagerflächen vorzusehen (nicht dargestellt).

In der beschriebenen Hochkantstellung kann der vorliegende Behälter 1 als bequeme und sichere Transportvorrichtung beispielsweise für gefüllte Baby- oder Saugflaschen verwendet werden. Er kann mit Hilfe des jeweiligs ausgeklappten Handgriffes 14 oder 15 einfach und gut hantiert werden, und er steht dank des angeklappten Handgriffs 15 oder 14 auf einer hinreichend ebenen Unterlage einwandfrei und stabil. Als weiterer Vorteil für diese Transportfunktion erweist sich beim Transportieren von mit erwärmter Nahrung gefüllten Flaschen die nicht unbedeutende Wärmeisolierwirkung des aus Kunststoff und damit aus relativ gut wärmeisolierendem Material bestehenden Behälters 1. Andererseits kann der Behälter 1 auch als Versandverpackung von einem Erzeuger zum Verkäufer sowie als Verkaufsverpackung beim Verkäufer verwendet werden, wobei der zumindest durchscheinende vorzugsweise glaskare Behälter bzw. wenigstens der transparente Deckel einen guten Einblick auf die im Behälter aufgenommen und zur Schau gestellten Waren erlaubt. Da der Behälter währenddessen immer dicht verschlossen bleibt, ist eine hygienische Abwicklung der Vorgänge: Versand-Verkauf gewahrleistet. Ebenso wird im Falle des Transports von gefüllten Flaschen durch den dichten Verschluß des Deckels 3 am Behälterkörper 2 eine saubere, hygienische Aufbewahrung der Flaschen sowie anderer Gegenstände sichergestellt.

An der dargestellten Vorrichtung sind sodann weitere Vorkehrungen getroffen, um sie für ihre dritte Funktion, als Desinfektionsvorrichtung, geeignet zu machen. So ist insbesondere ein gitterförmiger Rost 22 vorgesehen, der einen herausnehmbaren eigenen Bauteil bildet, und der sich in der in den Behälter 1 eingesetzten Lage (s. Fig. 2, 3 und 4) einerseits mit randseitigen Füßen 23 am Behälterboden 24 und andererseits in der Mitte des Behälterbodens 24 direkt auf eine zentrale Einbuchtung 25 des Behälterbodens 24 abstützt. Der Rost 22 ist dabei, um sein Einsetzen in den Behälter 1 problemlos zu gestalten, zumindest hinsichtlich zweier zueinander sowie zur Rostebene senkrechter Mittelebenen symmetrisch ausgebildet. Dieser Rost 22 dient beim Desinfizieren von Babyartikeln, wie Schnullern, Saugern, Saugflaschen, Beißringen und dergl. Gegenständen, als Distanzelement oder Distanzhalter, um diese Gegenstände im Abstand vom eigentlichen Behälterboden 24 zu tragen, wobei bis in die Höhe des Rostes 24 in den Behälter Wasser z.B. in einer Menge von ca. 100 ml, je nach Größe des Behälters und nach Volumen der zu desinfizierenden Gegenstände, eingegossen wird. Demgemäß tauchen die zu desinfizierenden Gegenstände nicht in den Wasservorrat ein, und sie werden in einem Mikrowellenfeld, mit dem das Wasser erhitzt und verdampft wird, in einem Sattdampf desinfiziert.

Hiefür wird der Deckel 3 auf den Behälterkörper 2 aufgesetzt und fixiert, nachdem wie erwähnt das Wasser in den Behälterkörper 2 eingebracht, danach der Rost 24 eingelegt und auf diesem Rost die Gegenstände placiert wurden. Die so verschlossene Vorrichtung samt Inhalt wird dann z.B. in einen im Haushalt vorhandenen Mikrowellenherd gebracht, an dem die erforderlichen Einstellungen hinsichtlich Leistung und Zeit vorgenommen werden. Das Mikrowellenfeld bringt das Wasser sodann sehr schnell zum Sieden und verdampft den größten Teil hiervon. Der Dampf steigt über den Gitterrost 12 auf, und er erfüllt den gesamten Behälter 1 und bewirkt die Desinfektion oder Dampfsterilisation.

Bei der beschriebenen Desinfektionsbehandlung im Mirkowellenherd werden zweckmäßigerweise die Handgriffe 14, 15 an den Behälterkörper angeklappt, so daß der Behälter 1 auch in verhältnismäßig kleinen Mikrowellenherden Platz findet. Nach dem Desinfektionsvorgang bieten die Handgriffe den Vorteil, daß sie das Herausnehmen der Vorrichtung aus dem Mikrowellenherd erleichtern, wobei auch zu berücksichtigen ist, daß der eigentliche Behälter samt Deckel nach dem Desinfektionsvorgang durch die Dampfentwicklung in der Regel heiß sein wird.

Um einen übermäßigen Druckaufbau im Behälterinneren zu verhindern und den überschüssigen Dampf aus den Behälter entweichen zu lassen, ist im Deckel 3 an der Oberseite eine Druckausgleichsöffnung 26 vorgesehen, die in der Ausführungsform gemäß Fig. 1 bis 4 in der Art eines Drucksicherheitsventils mit einem Verschlußkörper 27 versehen ist, der normalerweise unter dem Druck einer Feder 28, z.B. einer Schraubendruckfeder, die auf hakenförmige Fortsätze 29 des Verschließkörpers 27 einwirkt und sich mit dem anderen Ende am Deckel 3 abstützt, in Schließlage gehalten ist. Bei steigendem Innendruck im Behälter 1 bewegt sich der Verschließkörper 27 entgegen dem Druck der Feder 28 nach oben, wodurch ein Druckausgleich durch die Öffnung 26 hindurch erfolgen kann.

Um überprüfen zu können, daß die zu desinfizierenden Gegenstände ausreichend lange bei einer aureichend hohen Temperatur der Dampf-Desinfektionsbehandlung unterzogen wurden, ist sodann an der Innenseite des Deckels 3 an der vorderen Längswand 30 mittig ein Temperaturindikator als Temperaturanzeige 31 angebracht. Im einzelnen kann dieser Temperaturindikator, der z.B. plättchenförmig ausgebildet ist und auf Flüssigkristallbasis, mit zumindest einem sich verfärbenden Bereich, arbeitet und nachstehend einfach als Temperaturanzeigeplättchen bezeichnet wird, einfach in eine taschenartige Vertiefung 32 mit das Temperaturanzeigeplättchen 31 übergreifenden Randleisten 33 eingesetzt sein, wie aus Fig. 6 ersichtlich ist. Das Temperaturanzeigeplättchen 31 hat dabei vorzugsweise eine von der Zeit und Temperatur abhängige Anzeigecharkteristik, so daß ein Wechsel in der Farbe des aktiven Plättchenteils, z.B. von orange nach grün, was von außerhalb des Mikrowellenherdes gut beobachtet werden kann, die zufriedenstellende Beendigung des Desinfektionsvorgangs anzeigt.

An sich könnten selbstverständlich aber auch andere Temperaturanzeigeelemente angebracht werden, wie etwa herkömmliche Thermometer.

Zu erwähnten ist auch, daß als Druckausgleichsöffnungen anstatt der Öffnung 26 einfache (gegebenenfalls mehrere, möglichst klein bemessene) Öffnungen ohne Verschließkörper beispielsweise in der Deckeloberseite vorgesehen sein können, wie dies der Einfachheit halber in Fig. 3 schematisch bei 34 angedeutet ist. Gegebenenfalls können in diesen Öffnungen 34 auch teilweise durchlässige Folien oder Membranen oder Gewebe angebracht sein, die Staub und dergl. Fremdpartikel davon abhalten, in das Behälterinne zu gelangen, die jedoch soweit durchlässig sind, daß bei steigendem Druck, bei der Verdampfung von Wasser im Inneren des Behälters 1, ein Druckausgleich durch Dampf- bzw. Luftdurchtritt ermöglicht wird.

Wie aus Fig. 2 und 3 ersichtlich ist, ist der Behälterkörper 2 an seiner Unterseite mit Füßen 35 versehen, die durch entsprechende, etwa halbkugelförmige Ausbuchtungen des Behälterbodens 24 gebildet sind. Diese Füße 35 definieren die normale Standfläche des Behälters 1, und für eine Stapelung mehrerer Behälter übereinander, etwa bei der Zurschaustellung in Verkaufslokalen, kann der Behälterdeckel 3 an seiner Oberseite mit entsprechend halbkugelförmig ausgebildeten Vertiefungen 36 geformt sein, die im gestapelten Zustand mehrerer Behälter die Füße eines darüber befindlichen Behälters aufnehmen.

In Fig. 7 ist ein gegenüber der Ausführungsform von Fig. 2 modifizierter Rastverschluß des Deckels 3 mit dem Behälterkörper 2 im Bereich der Randflanschen 4 bzw. 5 veranschaulicht; dabei ist nun die Rastnase 12 an einer außen am Flansch 5 anschließenden vertikalen Griffplatte 37 vorgesehen, und diese Rastnase 12 untergreift im aufgeschnappten Zustand des Deckels 3 den Randflansch 4 des Behälterkörpers 2, wie aus der Darstellung in Fig. 7 hervorgeht. Um ein Lösen dieser Rastverbindung zwecks Abnahme des Deckels 3 vom Behälterkörper 2 zu erleichtern, können auch im Deckelflansch 5 benachbart der Griffplatte 27 Öffnungen 38 vorgesehen sein, die eine elastische Verformung in diesen Bereich, für ein Verschwenken der Griffplatte 37 im Gegenuhrzeigersinn, erleichtern.

Eine weitere Verschlußvorrichtung ist schematisch in Fig. 8 veranschaulicht. Dabei handelt es sich um einen Knebelverschluß mit einem hakenförmigen Verschlußhebel 39, der am Deckel 3 im Bereich von dessen Flansch 5, bei 40, schwenkbar angelenkt ist und den Randflansch 4 des Behälterkörpers 2 in der Schließlage untergreift.

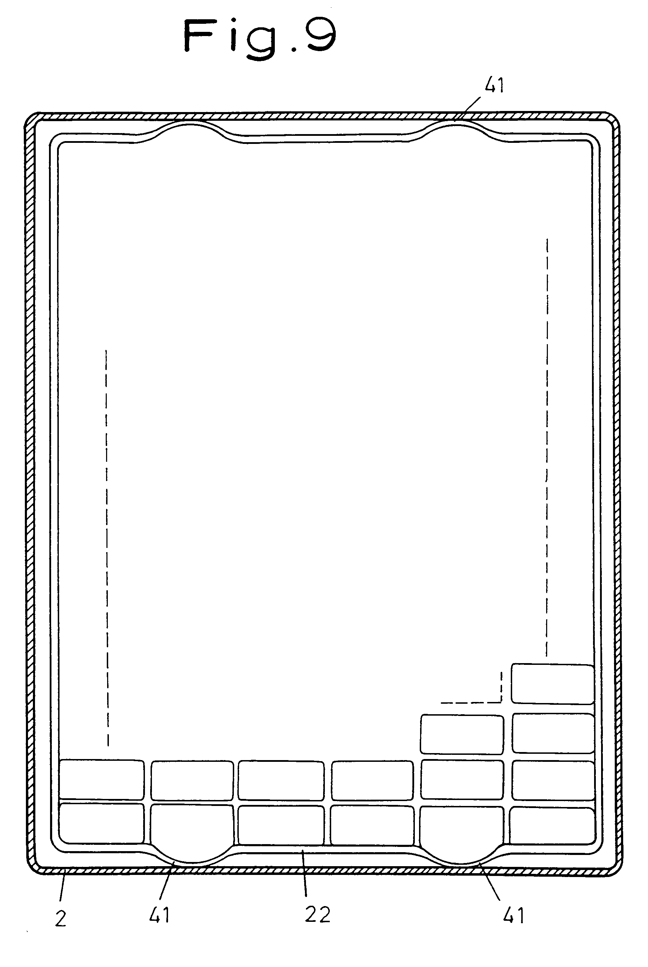

In Fig. 9 ist schließlich ein modifizierter Rost 22 in einer Draufsicht, in einer Position innerhalb des im Horizontalschnitt veranschaulichten Behälterkörpers 2, veranschaulicht, wobei ersichtlich ist, daß dieser Rost 22 gemäß Fig. 9 beispielsweise an seinen zwei einander gegenüberliegenden kürzeren Seiten mit elastisch verformbaren Rand-Ausbuchtungen 41 versehen ist, mit denen der Rost 22 unter Druck, nach elastischer Verformung, an den Innenflächen der Behälterkörperwände anliegt, so daß der Rost 22 auf diese Weise in seiner Lage relativ sicher festgehalten ist und nicht lose im Behälter 1 eingelegt ist. Nichtsdestoweniger ist eine problemlose Herausnahme des Rosts 22 auch in der Ausführungsform nach Fig. 9 möglich.

Selbstverständlich können solche elastisch deformierbaren Ausbuchtungen 41 - zusätzlich oder stattdessen - auch an den Längsseiten des Rostes 22 vorgesehen sein.

## Patentansprüche

1. Verpackungs-, Transport- und Desinfektionsvorrichtung für Babyartikel und dergl. Gegenstände, wie z.B. Saugflaschen, Sauger, Schnuller, gekennzeichnet durch einen aus einem mikrowellentauglichen Kunststoff, wie Polykarbonat, bestehenden Behälter (1) mit Deckel (3), wobei der Deckel (3) dicht mit dem oben offenen Behälterkörper (2) verbindbar ist, der Behälterkörper (2) an zwei einander gegenüberliegenden Seiten mit angelenkten Handgriffen (14, 15) versehen ist, von denen zumindest einer an den Behälterkörper (2) anklappbar ist, um eine Behälter-Standfläche zusätzlich zum Boden (24) des Behälters (1) vorzusehen, im Deckel (3) zumindest eine Druckausgleichsöffnung (26; 34) vorgesehen ist und der Behälter (1) mit einer von außen sichtbaren Temperaturanzeige (31) ausgerüstet ist.

2. Vorrichtung nach Anspruch 1, dadurch gekennzeichnet, daß zumindest der Deckel (3) aus klarem Polykarbonat besteht.

3. Vorrichtung nach einem Anspruch 1 oder 2, dadurch gekennzeichnet, daß der Behälter (1) annähernd quaderförmig ausgebildet ist.

4. Vorrichtung nach einem der Ansprüche 1 bis 3, dadurch gekennzeichnet, daß an den einander gegenüberliegenden Seiten des Behälterkörpers Scharniere für die Handgriffe (14, 15) angeformt sind.

5. Vorrichtung nach Anspruch 4, dadurch gekennzeichnet, daß für jeden Handgriff (14, 15) zwei Scharnierblöcke (16, 17) in Abstand voneinander am Behälterkörper (2) angeformt sind und diese Scharnierblöcke zapfenartige Angeln (18) des jeweiligen bügelartigen Handgriffs (14, 15) schwenkbar aufnehmen.

6. Vorrichtung nach Anspruch 4 oder 5, dadurch gekennzeichnet, daß die Scharniere jeweils direkt unterhalb des die obere Öffnung des Behälterkörpers (2) begrenzenden Randes vorgesehen sind.

7. Vorrichtung nach einem der Ansprüche 4 bis 6, dadurch gekennzeichnet, daß die Handgriffe (14, 15) in den Scharnieren zwischen zwei Raststellungen, einer an den Behälterkörper (2) angeklappten Stellung und einer rechtwinkelig hievon abstehenden Stellung, verschwenkbar gelagert sind.

8. Vorrichtung nach einem der Ansprüche 4 bis 6, dadurch gekennzeichnet, daß die Handgriffe (14, 15) mit Reibungspassung in den Scharnieren gelagert sind.

9. Vorrichtung nach einem der Ansprüche 1 bis 8, dadurch gekennzeichnet, daß die Handgriffe (14, 15) in der an den Behälterkörper (2) angeklappten Stellung bis annähernd zum Boden (24) des Behälterkörpers reichen.

10. Vorrichtung nach einem der Ansprüche 1 bis 9, dadurch gekennzeichnet, daß die Handgriffe (14, 15) selbst in der an den Behälterkörper (2) angeklappten Stellung mit ihrer Außenseite einen Teil der zusätzlichen Behälter-Standfläche bilden.

11. Vorrichtung nach einem der Ansprüche 1 bis 10, dadurch gekennzeichnet, daß im Behälter (1) am Boden (24) Distanzelemente zum Abstützen der zu desinfizierenden Gegenstände in Abstand vom Behälterboden vorgesehen sind.

12. Vorrichtung nach Anspruch 11, dadurch gekennzeichnet, daß die Distanzelemente durch einen herausnehmbaren, z.B. gitterartigen Rost (22) gebildet sind.

13. Vorrichtung nach Anspruch 12, dadurch gekennzeichnet, daß sich der Rost (22) mit randseitigen Füßen (23) am Boden (24) des Behälterkörpers (2) abstützt.

14. Vorrichtung nach Anspruch 12 oder 13, dadurch gekennzeichnet, daß der Rost (22) im Bereich der Mitte des Bodens (24) des Behälterkörpers (2) auf einer hochgezogenen Einbuchtung (25) des Bodens (24) aufliegt.

15. Vorrichtung nach einem der Ansprüche 12 bis 14, dadurch gekennzeichnet, daß der Rost (22) zumindest an zwei einander gegenüberliegenden Seiten an seinem Rand elastisch verformbare Ausbuchtungen (41) aufweist, mit denen er unter federndem Druck an den benachbarten Seitenwänden des Behälterkörpers (2) anliegt.

16. Vorrichtung nach einem der Ansprüche 12 bis 15, dadurch gekennzeichnet, daß der Rost (22) symmetrisch ausgebildet ist.

17. Vorrichtung nach einem der Ansprüche 1 bis 16, dadurch gekennzeichnet, daß der Behälterkörper (2) und der Deckel (3) mit zueinander passenden, rundum laufenden, nach außen abstehenden Flanschen (4, 5) für ihre dichte Verbindung versehen sind.

18. Vorrichtung nach einem der Ansprüche 1 bis 17, dadurch gekennzeichnet, daß als Temperaturanzeige (31) an bzw. in einer Wand (30) des transparenten Deckels (3) innen ein Temperaturanzeigeplättchen angebracht ist.

19. Vorrichtung nach Anspruch 18, dadurch gekennzeichnet, daß das Temperaturanzeigeplättchen in eine taschenartige Vertiefung (32) mit übergreifenden Randleisten (33) eingesetzt ist.

20. Vorrichtung nach Anspruch 18 oder 19, dadurch gekennzeichnet, daß das Temperaturanzeigeplättchen ein Temperaturindikator auf Flüssigkristallbasis ist.

21. Vorrichtung nach einem der Ansprüche 18 bis 20, dadurch gekennzeichnet, daß das Temperaturanzeigeplättchen eine ZeitTemperatur-abhängige Charakteristik hat und die Erreichung einer vorgegebenen, für eine einwandfreie Desinfektion notwendigen Temperatur über eine vorgegebene Zeitdauer anzeigt.

22. Vorrichtung nach einem der Ansprüche 1 bis 21, dadurch gekennzeichnet, daß der Deckel (3) mittels Schnapp-oder Knebelverbindungen (11, 12; 39) mit dem Behälterkörper (2) verbindbar ist.

23. Verfahren zur Desinfektion von Babyartikeln und dergl. Gegenstände, wie z.B. Saugflaschen, Sauger, Schnuller, unter Verwendung einer Vorrichtung nach einem der Ansprüche 1 bis 22, dadurch gekennzeichnet, daß in den Behälterkörper (2) Wasser eingebracht wird und die zu desinfizierenden Gegenstände zumindest im wesentlichen oberhalb des Niveaus des eingebrachten Wassers angebracht werden, und daß nach Schließen des Behälters (1) mit dem Deckel (3) die Vorrichtung einem Mikrowellenfeld ausgesetzt wird, um die Gegenstände durch eine Behandlung im dabei entstehenden Wasserdampf zu desinfizieren.

24. Verfahren nach Anspruch 23, dadurch gekennzeichnet, daß die Menge des eingebrachten Wassers ausreichend bemessen wird, so daß ein Wasserrest nach der Dampf-Desinfektionsbehandlung im Behälter (1) bleibt.

## Claims

1. A packaging, transporting and disinfecting device for baby articles and the like objects, such as, e.g., feeding bottles, nipples, pacifiers, characterised by a container (1) made of a synthetic material fit for use with microwaves, such as polycarbonate, and including a lid (3), wherein the lid (3) is sealingly connectable with the upwardly open container body (2), the container body (2) is provided with handles (14, 15) articulately fastened to two opposite sides, at least one of which handles is foldable at the container body (2) to provide a container base in addition to the bottom (24) of the container (1), at least one pressure relief vent (26; 34) is provided in the lid (3), and the container (1) is equipped with an externally visible temperature indicator (31).

2. A device according to claim 1, characterised in that at least the lid (3) is made of clear polycarbonate.

3. A device according to any one of claims 1 or 2, characterised in that the container (1) is designed to be approximately parallelepipedal.

4. A device according to any one of claims 1 to 3, characterised in that the oppositely located sides of the container body are formed with hinge connections for the handles (14, 15).

5. A device according to claim 4, characterised in that the container body (2) is formed with two spaced apart hinge blocks (16, 17) for each handle (14, 15), which hinge blocks pivotably receive the pin-like hinges (18) of the respective shackle-shaped handle (14, 15).

6. A device according to claim 4 or 5, characterised in that the hinge connections each are provided directly below the edge delimiting the upper opening of the container body (2).

7. A device according to any one of claims 4 to 6, characterised in that the handles (14, 15) are mounted in the hinge connections so as to be pivotable between two locked positions, i.e., a position folded at the container body (2) and a position projecting therefrom at a right angle.

8. A device according to any one of claims 4 to 6, characterised in that the handles (14, 15) are mounted in the hinge connections with frictional engagement.

9. A device according to any one of claims 1 to 8, characterised in that the handles (14, 15) approximately reach as far as to the bottom (24) of the container body in the position folded at the container body (2).

10. A device according to any one of claims 1 to 9, characterised in that the handles (14, 15) themselves form part of the additional container base by their external sides when folded at the container body (2).

11. A device according to any one of claims 1 to 10, characterised in that spacer elements are provided on the bottom (24) in the container (1) for supporting the articles to be disinfected at a distance from the container bottom.

12. A device according to claim 11, characterised in that the spacer elements are formed by a removable, e.g. grid-like, grate (22).

13. A device according to claim 12, characterised in that the grate (22) is supported on the bottom (24) of the container body (2) by end feet (23).

14. A device according to claim 12 or 13, characterised in that the grate (22) rests on a raised indentation (25) of the bottom (24) in the region of the centre of the bottom (24) of the container body (2).

15. A device according to any one of claims 12 to 14, characterised in that the grate (22), at least on two oppositely located sides, comprises elastically deformable bulges (41) on its periphery, by which it contacts the neighbouring side walls of the container body (2) under resilient pressure.

16. A device according to any one of claims 12 to 15, characterised in that the grate (22) is symmetrically designed.

17. A device according to any one of claims 1 to 16, characterised in that the container body (2) and the lid (3), for their tight connection, are provided with complementary peripheral flanges (4, 5) projecting outwards.

18. A device according to any one of claims 1 to 17, characterised in that a temperature indication platelet is provided internally on or in a wall (30) of the transparent lid (3) as said temperature indicator (31).

19. A device according to claim 18, characterised in that the temperature indication platelet is inserted in a pocket-like depression (32) with overlapping shoulders (33).

20. A device according to claim 18 or 19, characterised in that the temperature indication platelet is a temperature indicator based on liquid crystals.

21. A device according to any one of claims 18 to 20, characterised in that the temperature indication platelet has a time-temperature-dependent characteristic, indicating the attainment of a predetermined temperature necessary for perfect disinfection over a predetermined period of time.

22. A device according to any one of claims 1 to 21, characterised in that the lid (3) is connectable with the container body (2) by means of a snap-in or toggle joint connection (11, 12; 39).

23. A method of disinfecting baby articles and the like objects, such as, e.g., feeding bottles, nipples, pacifiers, by using the device according to any one of claims 1 to 22, characterised in that water is introduced into the container body (2), the articles to be disinfected are arranged at least substantially above the level of the introduced water, and, upon closure of the container (1) by the lid (3), the device is exposed to a microwave field in order to disinfect the articles by treatment with the steam forming.

24. A method according to claim 23, characterised in that the amount of water introduced is sufficiently dimensioned such that residual water will remain in the container (1) after the steam disinfection treatment.

## Revendications

1. Dispositif d'emballage, de transport et de désinfection d'articles de puériculture et d'objets similaires, tels que biberons, tétines, sucettes, caractérisé par un récipient (1) en matière plastique résistant aux microondes, telle que du polycarbonate, avec couvercle (3), le couvercle (3) pouvant être assemblé de manière étanche au corps (2) du récipient, ouvert à sa partie supérieure, le corps (2) du récipient étant pourvu, sur deux côtés opposés l'un à l'autre, de poignées (14, 15) articulées, dont l'une au moins peut être rabattue contre le corps (2) du récipient, afin de prévoir une surface d'appui au récipient, supplémentaire au fond (24) du récipient (1), un orifice de compensation de pression (26 ; 34) étant prévu au moins dans le couvercle (3) et le récipient (1) étant équipé d'un indicateur de température (31) visible de l'extérieur.

2. Dispositif selon la revendication 1, caractérisé en ce qu'au moins le couvercle (3) est en polycarbonate clair.

3. Dispositif selon la revendication 1 ou 2, caractérisé en ce que le récipient (1) est approximativement parallélépipédique.

4. Dispositif selon l'une des revendications 1 à 3, caractérisé en ce que sur les côtés opposés l'un à l'autre du corps du récipient sont formées des charnières pour les poignées (14, 15).

5. Dispositif selon la revendication 4, caractérisé en ce que pour chaque poignée (14, 15) deux blocs de charnières (16, 17) sont formés à distance l'un de l'autre sur le corps (2) du récipient et ces blocs de charnières logent, de manière pivotante, des gonds (18) à la manière de pivots, de la poignée (14, 15) respective en forme d'étrier.

6. Dispositif selon la revendication 4 ou 5, caractérisé en ce que les charnières sont prévues chacune directement au-dessous du bord limitant l'ouverture supérieure du corps (2) du récipient.

7. Dispositif selon l'une des revendications 4 à 6, caractérisé en ce que les poignées (14, 15) sont montées de manière à pouvoir pivoter dans les charnières, entre deux positions d'arrêt, une position rabattue contre le corps (2) du récipient et une position faisant saillie à angle droit de la première.

8. Dispositif selon l'une des revendications 4 à 6, caractérisé en ce que les poignées (14, 15) sont montées dans les charnières avec ajustement à friction.

9. Dispositif selon l'une des revendications 1 à 8, caractérisé en ce que les poignées (14, 15) en position rabattue contre le corps (2) du récipient, s'étendent à peu près jusqu'au fond (24) de celui-ci.

10. Dispositif selon l'une des revendications 1 à 9, caractérisé en ce que les poignées (14, 15) forment elles-mêmes, dans la position rabattue contre le corps (2) du récipient, avec leur côté extérieur, une partie de la surface d'appui supplémentaire du récipient.

11. Dispositif selon l'une des revendications 1 à 10, caractérisé en ce que dans le récipient (1), sur le fond (24), sont prévus des éléments d'écartement destinés à soutenir les objets à désinfecter, à distance du fond du récipient.

12. Dispositif selon la revendication 11, caractérisé en ce que les éléments d'écartement sont formés par une grille (22) amovible, par exemple à barreaux entrecroisés.

13. Dispositif selon la revendication 12, caractérisé en ce que la grille (22) prend appui sur le fond (24) du corps (2) du récipient, par des pieds (23) situés sur les bords.

14. Dispositif selon la revendication 12 ou 13, caractérisé en ce que la grille (22) repose, dans la zone du milieu du fond (24) du corps (2) du récipient, sur une surélévation (25) du fond (24).

15. Dispositif selon l'une des revendications 12 à 14, caractérisé en ce que la grille (22) présente, au moins sur deux côtés opposés l'un à l'autre, sur son bord, des creux (41) élastiquement déformables, par lesquels elle s'applique, sous une pression élastique, contre les parois latérales voisines du corps (2) du récipient.

16. Dispositif selon l'une des revendications 12 à 15, caractérisé en ce que la grille (22) est symétrique.

17. Dispositif selon l'une des revendications 1 à 16, caractérisé en ce que le corps (2) du récipient et le couvercle (3) sont pourvus de brides (4, 5) adaptées l'une à l'autre, suivant tout le pourtour, faisant saillie vers l'extérieur, pour leur assemblage étanche.

18. Dispositif selon l'une des revendications 1 à 17, caractérisé en ce qu'une plaquette d'indication de température est placée à l'intérieur sur ou dans une paroi (30) du couvercle (3) transparent, pour servir d'indicateur de température (31).

19. Dispositif selon la revendication 18, caractérisé en ce que la plaquette d'indication de température est placée dans un creux (32) en forme de poche avec des listels de bordure (33) passant par dessus.

20. Dispositif selon la revendication 18 ou 19, caractérisé en ce que la plaquette d'indication de température est un indicateur de température à base de cristaux liquides.

21. Dispositif selon l'une des revendications 18 à 20, caractérisé en ce que la plaquette d'indication de température présente une caractéristique dépendant du temps et de la température et indique qu'une température donnée, nécessaire pour une désinfection parfaite, est atteinte pendant un intervalle de temps donné.

22. Dispositif selon l'une des revendications 1 à 21, caractérisé en ce que le couvercle (3) peut être assemblé au corps (2) du récipient par des liaisons à encliquetage ou à manettes (11, 12 ; 39).

23. Procédé de désinfection d'articles de puériculture et d'objets similaires, par exemple biberons, tétines, sucettes, par utilisation d'un dispositif selon l'une des revendications 1 à 22, caractérisé en ce que de l'eau est introduite dans le corps (2) du récipient et les objets à désinfecter sont placés au moins pour l'essentiel au-dessus du niveau de l'eau introduite, et en ce qu'après fermeture du récipient (1) avec le couvercle (3), le dispositif est soumis à un champ de micro-ondes, afin de désinfecter les objets par traitement dans la vapeur d'eau qui se forme ainsi.

24. Procédé selon la revendication 23, caractérisé en ce que la quantité d'eau introduite est suffisante pour qu'il reste de l'eau dans le récipient (1), après traitement de désinfection à la vapeur.
